# EUROPEAN PATENT APPLICATION

(11) **EP 4 181 151 A1**
(43) Date of publication of application: **17.05.2023**
(21) Application number: 21207732.5
(22) Date of filing: 11.11.2021
(51) Int. Cl.: G16H 20/70

(54) **A SYSTEM AND METHOD FOR ASSISTING THE DEVELOPMENT OF AN ADULT-CHILD ATTACHMENT RELATIONSHIP**

(71) Applicant: Koninklijke Philips N.V., 5656 AG Eindhoven (NL)
(72) Inventor: WESTERINK, Joanne Henriëtte Desirée Monique, Eindhoven (NL); LEMMENS, Paul Marcel Carl, Eindhoven (NL); LEUFKENS, Timmy Robertus Maria, Eindhoven (NL); MØST, Elsa Inger Stapel, Eindhoven (NL); VAN WISSEN, Arlette, Eindhoven (NL); DE VREEDE, Jasper, Eindhoven (NL); HAEX, Nicole Petronella Martien, Eindhoven (NL); TIEMANN, Christian Andreas, Eindhoven (NL); OTTE, Renée Antoinette, Eindhoven (NL); WOUTERS, Cornelis Bernardus Aloysius, Eindhoven (NL); HAMELMANN, Paul Christoph, Eindhoven (NL); DENISSEN, Adrianus Johannes Maria, Eindhoven (NL)
(74) Representative: Philips Intellectual Property & Standards

(57) **Abstract**

A system is provided for assisting the development of an adult-child attachment relationship. One or more child physiological or behavioral signals are sensed for the adult and the child. An adult-child activity is chosen by the system and an association between the adult and child signals is analyzed so as to estimate a level of synchrony between the adult and child associated with the selected adult-child activity. An output is generated which depends on the level of synchrony.

## Description

### FIELD OF THE INVENTION

This invention relates to the emotional bond between an adult, such as a parent, relative or other carer, and a child. This emotional bond will be termed an adult-child attachment relationship in this document. The invention relates to a system and method for assisting adults, such as parents, in improving this attachment relationship.

### BACKGROUND OF THE INVENTION

Literature on the development of infants and children is very clear that it is important for the child to have a good, secure attachment to one or more parents. This benefits the mental development of the infant and also has a beneficial impact much later in childhood and adulthood. For this reason, young parents can be assumed to be interested in developing a secure attachment relationship with their infant. And in addition of course, such a relationship is also a source of pleasure in itself.

After birth, and sometimes even before, the relationship between a parent and an infant develops during interaction between them. It takes the form of co-regulation: the parent influences the actions of the child, and the child influences the actions of the parent. This already begins in a very physiological way, where skin-to-skin contact with the mother leads to lower respiration rates of the baby.

For this reason kangaroo care, when the newborn baby is carried close to the parent's body, is considered a great way of building attachment. The relationship gradually develops into behavioral aspects when the parent (or other adult) and child attend and react to each other. One clear situation is when the child is crying for comfort, and the parent indeed reacts to the child, trying to comfort it. Another situation occurs in playful interaction, for instance when the child and adult are laughing at the same moments in time, or looking at the same object at the same moment in time, or showing emotions (e.g. affect, joy) at the same moment in time.

These behaviors are coupled and therefore show synchrony. It has been reported that the level of behavioral synchrony exhibited is predictive of whether a secure attachment relationship will result or not, for example as reported in Jaffe, J., Beebe, B., Feldstein, S., Crown, C. L., Jasnow, M. D., Rochat, P., & Stem, D. N. (2001). Rhythms of dialogue in infancy: Coordinated timing in development. Monographs of the society for research in child development, i-149.

Further research has shown that the importance of parent-child synchrony does not only hold for various aspects of behavior, but that there are also indications for the relevance of synchrony in physiological parameters. For example, synchrony has been found in heart rate (HR) when mothers and their pre-school children are viewing low-action videos together, but this synchrony is absent when the child is maltreated.

It can therefore be concluded that synchrony in behavior as well as in physiology may be used as a marker of the development of a good attachment relationship.

While young parents are typically interested in developing a secure attachment relationship with their child as mentioned above, it might not always be easy. One of the reasons might be that in our individualistic society, young parents are more often at work and receive less of an example from others (family members) around them.

It would therefore be of interest to have a system, such as an application to be provided on an existing portable device, that can inform the adult whether they are indeed building such a secure relationship. Since people, children and parents are not all the same, the approach ideally will not be "one-size fits all" either, but needs to be personalized.

### SUMMARY OF THE INVENTION

The invention is defined by the claims.

According to examples in accordance with an aspect of the invention, there is provided a system for assisting in the development of an adult-child attachment relationship, comprising:
a physiological or behavioral sensor arrangement for sensing one or more child physiological or behavioral signals and for sensing one or more adult physiological or behavioral signals;
an output system; and
a controller,
wherein the controller is adapted to:
   for one of a set of possible adult-child activities, analyze an association between the adult physiological or behavioral signals and the child physiological or behavioral signals, and thereby estimate a level of synchrony between the adult and child associated with the selected adult-child activity; and
   control the output system to provide an output which depends on the level of synchrony.

The controller is for example adapted to:
select said one of the set of possible adult-child activities and control the output system to identify a selected adult-child activity to be performed; or
receive an indication of a user-selected adult-child activity;

Thus, the system may propose an activity to the user or it may be told what activity the user has chosen.

The system may for example initially propose an activity for example based on what normally works for a child that age. The system automatically assesses the synchrony between an adult and a child during an activity, and thus it can learn over time to suggest activities to improve the synchrony between the adult and child, personalized to that adult-child relationship. Thus, it provides assistance to adults, typically the parents of the child, to help them learn how to form an attachment relationship more effectively with their child, by engaging in specific activities at the most appropriate times.

The synchrony may be measured as a simple level of correlation between the physiological or behavioral signals, or more complex measures may be used. For example, any measure of similarity between two signals may be used. The comparison may be in the time domain or in the frequency domain. A covariance is another example of a suitable comparison measure. The level of synchrony is indicative of the adult-child attachment relationship.

The physiological parameters may be vital signs signals, and the behavioral signals may for example comprise signals which monitor responses such as facial expressions, sounds (laughter, crying etc.).

The output which depends on the level of synchrony may for example simply be an indication of the actual (estimated) level of synchrony that has been achieved by that activity, to enable the adult to learn about the effectiveness of different activities.

The system may give tips to adults as to what they could try in order to achieve improved synchrony and hence a stronger attachment relationship. The tips may also aim at increasing a particular level of synchrony more quickly. By way of example there are two different types of tips the system could give, such as:
(i) An invitation to try a particular activity (e.g. "we advise the peekaboo activity because it works well for other children that age");
(ii) An invitation to adapt behavior while doing a specific activity that does not bring optimal results yet (e.g. when the peekaboo activity does not lead to high synchrony: "you could try waiting a bit longer before you say peekaboo and remove the hands from your face".

The output system may comprise different types of device, for example a display for indicating an activity, and other devices for providing feedback which depends on the synchrony level.

The invention thus provides a system that can give parents personalized advice on what to do in order to support the build-up of a secure attachment relationship. This may be of particular interest directly after birth, and when the child needs comforting.

The system may comprise a memory, or a communications system for interrogating an external memory, storing the set of possible adult-child interactive activities and historical data relating to the use of the adult-child interactive activities, and wherein the controller is adapted to select one of a set of possible adult-child activities based in part on the historical data and to control the memory to record the level of synchrony as historical data.

The system thus can propose an activity for example based on what normally works for a child that age, or based on what has worked for this particular adult-child combination in the recent past using the historical data.

The historical data may include data about other adult-child relationships, so that the present adult-child relationship can be compared with that of other adults (parents). Thus, the system can learn that generally for a child of the particular age and with this particular level of synchrony, particular activities are found to be effective. This will for example require the consent of the adult to use the uploaded data for the improvement of the system as a whole.

The historical data for example records which adult tried the activity, and what was the resulting level of synchrony. The historical data may for example comprise individual histories for both parents: one for the child and mother and one for the child and father. The father may for example be really bad at playing peekaboo, while the mother is very good at it. The historical data for example also records when the activity was tried.

The controller is for example adapted to select the one or more adult-child activities taking account of the age of the child. Different activities will be appropriate for different ages, and the recommendations by the system can thus evolve over time.

The child and adult physiological or behavioral signals may each comprise or identify one or more of:
heart rate;
skin conductance;
respiration characteristics;
direction of gaze;
type of facial expression;
sounds made by the child and/or adult;
movements.

Thus, various different signals may be measured. The heart rate may for example be monitored by a PPG sensor. Facial expression and movements may be monitored by a camera system. Some of these measures are for example for measuring stress levels, indicative of child distress. Indeed, a stress level may be derived and presented to the user as another output.

The same or different physiological or behavioral signals may be measured for the adult as for the child, and an association can also be assessed between different types of signal. For example, it is also possible to determine the synchrony of two signals of different types, e.g. between the heart rate of the child, and the skin conductance of the adult.
However, preferably the same signal is measured for the adult and the child.

In all cases, the correlation or other comparison result between the two measured signals is indicative of the process of establishing a relationship between the adult and child.

The system may comprise a microphone for recording the child and adult "physiological or behavioral signals" in particular in the form of sounds made during adult-child interaction, which are thus behavioral signals. Filtering may be used to separate the adult sounds from the child sounds, so that the association between them can be assessed.

Other examples of signals which may be measured are heart rate using a camera, for example from the faces of the adult and child at the same time. Filtering again may be used to separate out the adult face (s) from the child face.

The controller may be adapted to derive a level of anxiety of the child from the child physiological or behavioral signal and optionally also a level of anxiety of the adult from the adult physiological or behavioral signal. It is thereby possible to determine if e.g. a parent's attempts to comfort a child are working.

The set of adult-child activities for example include one or more of:
a peek-a-boo game;
a sound copying game;
a Kangaroo care session;
the adult singing to the child;
the adult rubbing the child's back;
bedtime rituals such as bathing and story-telling.

These are all relatively short duration activities. Longer duration activities may also be stored, such as:
visiting a petting zoo or other venue for an interactive experience;
performing arts and crafts together, such as making a painting.

Various different activities may be used to promote improvement of the adult-child attachment relationship. The system may learn over time which activities show the best result for the particular adult-child relationship. The system may also learn that some activities have ceased to be effective, because child has grown older. in that they no longer yield such high synchrony levels the set may also evolve over time, with the user of the system having the option to add their own activities.

The controller may be adapted to provide a feedback signal which depends on the estimated level of synchrony, wherein the feedback signal identifies suitable activities for improving the level of synchrony. Thus, in addition to identifying activities, the system can provide more general advice.

The system may comprise a speaker for playing music, wherein the controller is adapted to select characteristics of the music in dependence on the estimated level of synchrony during the adult-child activity, such that the music reflects the level of synchrony to some extent. Thus, the music may be considered to be the "output which depends on the level of synchrony" which is provided by the output system.

Alternatively or additionally, the system may comprise a speaker for playing a story telling, wherein the controller is adapted to select the characteristics of the story telling voice in dependence on estimated level of synchrony, such that the story telling reflects the level of synchrony to some extent. Thus, the story telling audio may be considered to be the "output which depends on the level of synchrony" which is provided by the output system.

Music characteristics, for instance corresponding to a level of quality, are thus used as a feedback signal to subconsciously promote building of the adult-child attachment relationship. When the synchrony and hence the adult-child attachment relationship improves, the music quality improves (for the adult and/or the child), and in subconsciously trying to achieve a better music quality, parents then implicitly also improve the synchrony level.

This provides an automated way to improve synchrony levels without requiring active and deliberate effort by the adult.

The main function of the device in this particular example is thus not to give advice or propose a best new activity, but to provide an implicit closed feedback loop to optimize the synchrony during the activity that is being undertaken.

Over time, the adult will learn that when they perform certain activities (e.g. a peekaboo game) that improves the synchrony between the adult and child, the quality of the music will improve. Thus, the system continuously gives feedback as to whether the current activity works or not.

The feedback is implicit in that the music does not give a numeric assessment of the level of synchrony, but has characteristics which do depend on it. The music provides an implicit indication of synchrony level.

Musical degradation can be used that also the child can hear, e.g. by also degrading high musical frequencies.

A story telling quality can also be used as a feedback signal to subconsciously promote building of the adult-child attachment relationship. When the level of synchrony improves, the story telling quality improves (for the child) for example based on the timbre of the voice.

Other feedback examples are room illumination patterns or the creation of images, again with different characteristics, such as degraded quality, at low synchrony level.

The controller may be adapted to:
receive an identification of an adult-child activity to add to the set; or
request identification of an adult-child activity based on continuous monitoring of the synchrony level.

An adult e.g. parent can also create activities of their own. For example, an adult could be interested to find out if "dancing together" leads to high synchrony levels. The adult could then name and label such an activity, after which it is added to the list of potential activities.

The adult may decide not to follow a recommendation of the system and perform their own activity, but then learn that a high level of synchrony resulted. In this case, the adult can add the activity to those monitored by the system, and indeed the system can request that the activity that was being performed is added to the set.

The invention also provides a baby carrier which incorporates the system defined above.

The invention also provides a computer-implemented method for assisting the development of an adult-child attachment relationship, comprising:
during one of a set of possible adult-child activities, receiving one or more sensed child physiological or behavioral signals;
receiving or more adult physiological or behavioral signals;
analyzing an association between the adult physiological or behavioral signals and the child physiological or behavioral signals and thereby estimating a level of synchrony between the adult and child associated with the selected adult-child activity; and
controlling the output system to provide an output which depends on the level of synchrony.

The method may comprise:
(automatically) selecting said one of the set of possible adult-child activities and controlling the output system to identify a selected adult-child activity to be performed; or
receiving an indication of a user-selected adult-child activity.

Selecting one of a set of possible adult-child activities may further take account of historical data relating to the use of the adult-child activities and the method comprises recording the estimated level of synchrony as historical data.

The method may also comprise selecting one or more adult-child activities taking account of the age of the child.

The invention also provides a computer program comprising computer program code means which is adapted, when said program is run on the controller of the system defined above to implement the method defined above.

The invention also provides a controller configured to run the computer program.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for assisting the development of an adult-child attachment relationship; and
Figure 2 shows a computer-implemented method for assisting the development of an adult-child attachment relationship.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for assisting the development of an adult-child attachment relationship. One or more child physiological or behavioral signals are sensed for the adult and the child. An adult-child activity is chosen by the system and an association between the adult and child signals is analyzed so as to estimate a level of synchrony between the adult and child associated with the selected adult-child activity. An output is generated which depends on the level of synchrony.

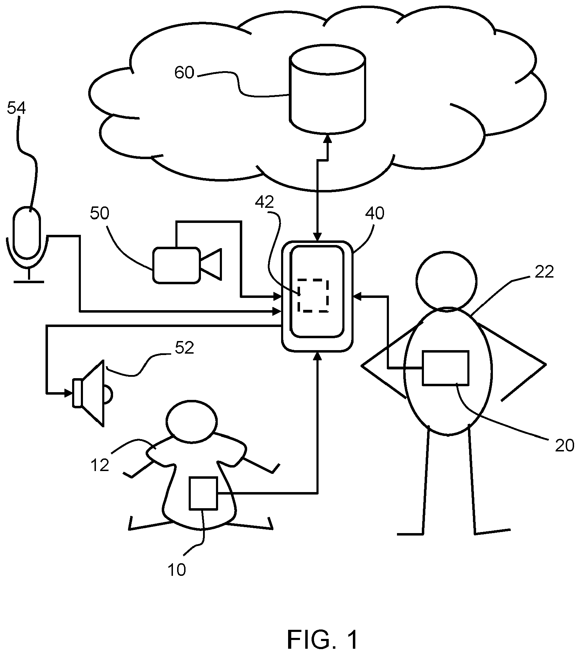

Figure 1 shows a system for assisting the development of an adult-child attachment relationship.

A physiological or behavioral sensor arrangement is provided for sensing one or more child physiological or behavioral signals and for sensing one or more adult physiological or behavioral signals. A first sensor 10 of the sensor arrangement is shown worn by the child 12 for sensing the one or more child physiological or behavioral signals, and a second sensor 20 of the sensor arrangement is shown worn by the adult 22 for sensing the one or more adult physiological or behavioral signals. The adult is typically a parent, but it could be a relative or other carer.

The signals are measurements of physiological or behavioral responses of the adult and child.

The sensor arrangement may additionally or alternatively comprise a sensor which monitors the child and/or the adult, such as camera 50 and a microphone 54.

The system has an output system 40 which in the example shown comprises a portable device of the adult, such as a mobile phone or tablet. In the case of a mobile phone, it may function as the output system with a display, as well as a speaker, but it may also function as the sensors of an input system, providing the microphone and camera.

A controller 42 is shown as part of the phone or tablet for processing the sensor data in the manner explained below, and for generating an output for the user (i.e. the adult). The controller 42 may of course be separate to the output device 40.

The camera 50 enables monitoring of the child and/or adult. In Figure 1, the camera is shown as a separate unit, for example monitoring a room in which the child and adult engage in interactive activities. The camera 50 may instead be the camera of the mobile phone or tablet 40 as mentioned above.

A speaker 52 enables audio output to be provided to the child and adult. In Figure 1, the speaker is shown as a separate unit. Again, the speaker 52 may instead be the speaker of the mobile phone or tablet 40 as mentioned above.

A microphone 54 enables monitoring of the sounds made by the child and/or adult. In Figure 1, the microphone 54 is shown as a separate unit. Again, the microphone may instead be the microphone of the mobile phone or tablet 40 as mentioned above.

A memory 60 stores a set of possible adult-child interactive activities, and preferably also historical data relating to the use of the adult-child interactive activities. The memory may be part of the system owned by the user, for example the memory of the portable device 40, or else the memory may be remote, such as a database accessed by the portable device 40 in the cloud. This latter example is shown in Figure 1.

The historical data for example records which adult tried the activity, and what was the resulting level of synchrony. The historical data may for example comprise individual histories for both parents: one for the child and mother and one for the child and father. There may also be separate historical data for different children of a family. The historical data for example also records when the activity was tried.

The set of adult-child activities for example include one or more of:
a peek-a-boo game;
a sound copying game;
a Kangaroo care session;
the adult singing to the child;
the adult rubbing the child's back;
bedtime rituals such as bathing and story-telling;
visiting a venue for an interactive experience; and
performing arts and crafts together.

However, activities may be added to the set by the user of the system.

The controller 42 selects one of the set of possible adult-child activities, and controls the output system to identify the selected adult-child activity to the user. This selection is for example based on the age of the child, and preferably the historical data.

While the activity is being performed, an association is analyzed between the adult physiological or behavioral signals and the child physiological or behavioral signals. In this way, a level of synchrony between the adult and child can be estimated associated with the selected adult-child activity. The correlation (or other similarity measure between the different signals) is a measure of the synchrony between the sensed physiological or behavioral signal of the adult and the sensed physiological or behavioral signal of the child. The output system may then provide an output which depends on the level of synchrony. The level of synchrony is also stored as new historical data.

Various different types of output may be generated which depend on the level of synchrony. A most basic example is simply to output to the user an indication of the level of synchrony that has been achieved. However, more elaborate approaches, described below, provide feedback for improving the level of synchrony in an automated manner. In addition. tips may be provided to the adult as to what they could try in order to achieve improved synchrony and hence a stronger attachment relationship. The tips may also aim at increasing a particular level of synchrony more quickly. Physiological patterns may be identified suggesting that the attachment relationship needs improvement and the system may provide advice to seek assistance.

The system may for example initially propose an adult-child activity based on what normally works for a child that age. The system can then learn over time to suggest activities to improve the synchrony between the adult and child, personalized to that particular adult-child relationship. Thus, over time, activities can be proposed based on what has worked for this particular adult-child combination in the recent past using the historical data. Also, one option is that the system might find out that child is relatively highly developed for its age, for instance because it already shows synchrony for activities for which children of the same age do not yet show synchrony. A similar conclusion may follow when the child does not show synchrony any more for activities for which other children of the same age do still show synchrony. In that case, the system could adapt to also propose other activities relatively early, because this child apparently is developing faster than the average child.

Thus, the system provides assistance to adults, typically the parents of the child, to help them learn how to form an attachment relationship more effectively with their child, by engaging in specific activities at the most appropriate times. The attachment relationship is the emotional relationship between the adult and child, and it has been found that the level of synchrony (correlation) between physiological and behavioral signals of the adult and child is indicative of the strength of the adult-child attachment relationship.

The physiological signals may be vital signs signals, and the behavioral signals may for example involve identification of facial expressions, sounds (laughter, crying etc.).

For example, the first and second sensors 10, 20 may include one or both of a PPG sensor for monitoring a heart rate (or heart rate variability) or respiration characteristics, and a skin conductivity sensor for monitoring skin conductance. Processing of the images captured by the camera 50 for example enable detection of a direction of gaze, a type of facial expression, movements and also heart rate by means of a vital signs camera. The various physiological parameters for example enable stress levels to be measured, indicative of child distress.

An overall synchrony level may be derived from multiple signals obtained at the same time, e.g. measured sound/speech, laughter, gaze direction and heart rate of both adult and child could be monitored while they are playing a game on a portable device.

The historical data may include data about other adult-child relationships, so that the present adult-child relationship can be compared with that of other adults and children.

The system tracks the historical synchrony levels for each activity over time as mentioned above. For example, for the average child a peekaboo game might bring high synchrony levels roughly between 5 and 8 months or 6 to 12 months, depending on the individuals. When the child turns 5 months, the adults are for example proposed to try this activity. If it works, it is proposed more often as a result of the historical data. However, if it does not work, it might be proposed again when the child is 6 months old, and maybe it will work then.

An activity will be proposed to the adult as long as it works well. Thus, for a particular child, if the peekaboo game has worked well even when the child is already 8 months old, the system will not stop advising this activity just because for most other children it does not work at this age. The individual child history is thus taken into account.

The same or different physiological or behavioral signals may be measured for the adult as for the child. It is most straightforward to determine a level of synchrony between the same types of signal. However, an association can also be assessed between different types of signal, for example to determine the synchrony between the heart rate of the child, and the skin conductance of the adult.

The signals may for example be measured by:
(i) the same device (e.g. 1 camera that measures the heart rate of both adult and child from the image);
(ii) two similar devices (e.g. adult and child wearing a similar PPG wrist band, delivering a heart rate for each); or
(iii) two devices of different types (e.g. measuring child heart by means of a camera, and adult heart rate by means of a wristband).

In all cases, the correlation between the two measured signals will be indicative of the process of establishing a relationship between the adult and child.

Some specific implementation examples will now be discussed.

### Example 1

A basic example may be based on heart rate measurements. These could for instance be taken when the child is brought to bed by a parent, for example during bedtime rituals, which may involve bathing and story-telling. For the child, the heart rate measurements could be derived from a vital signs camera directed at the bed, namely a remote PPG sensor, while the heart rate of the parent may be measured via a smartwatch (with a contact PPG sensor). Both signals are transmitted to and received by the controller 42 of the system, and the heart rate synchrony is derived by assessing to what extent the heart rates of the parent and child go up and down at the same moment in time. This could for instance be done by means of a simple correlation.

When the parent brings the child to bed, the parent can select one activity from a list of options presented by the system. One example of such an activity could be a peek-a-boo game. When parent and child engage in this activity, their synchrony is measured. The list of options that the system proposes is initially determined on the basis of what works well in general for various types of parents and children of that age. The system records which activities parents have undertaken with their child, when they did so, and which levels of synchrony were attained.

As more activities have been tried and their synchrony levels measured, the system may then adapt the proposed activities to include what recently yielded the highest synchrony levels between this particular parent and child. High synchrony levels are an indication that the co-regulation process is adapting well. Thus, the system helps the parents to undertake those activities that have a positive contribution to building a secure attachment relationship.

### Example 2

Another option is to make use of sound recordings by the microphone of the portable device 40, made while parent and child are playing games together on the couch.

Software on the portable device may be used to separate the sounds made by the parent from those made by the child, and assess to what extent the voiced and silent periods of both are in sync. The resulting level of synchrony can be monitored more or less in real time. If the parent sees that the activity of the last few minutes (e.g. a building block game) has resulted in high synchrony levels, they can store the recording, and give it a name (if the activity is not yet an option in the list of adult-child activities).

This activity will then become part of the list that is proposed by the system to the parent at future occasions. The synchrony levels attained for this particular game might rise on later occasions when the parent and child become more attuned to each other. But at some point, they might also drop, when the activity gets less and less appropriate for the stage of mental development of the child, and then the game is not so often proposed any more by the system.

### Example 3

In the examples above, the estimated synchrony level is used to adapt the (future) recommendations to the user, and the output may simply be an indication of the level of synchrony that is achieved. It is also possible to present the measured synchrony level as implicit feedback in quasi-real-time fashion, for instance considering the signals in the past 30 seconds.

The implicit feedback presentation may for example take the form of music, and this may be used to enable an automated (closed-loop) optimization of the synchrony level: The quasi-real time synchrony signal is fed back to parent and child in such a way that it rewards them if the synchrony is high, e.g. playing high quality music for high synchrony levels and (slightly) degraded music quality for low synchrony levels. The music then reflects the level of synchrony to some extent, as the characteristics of the music are adapted in dependence on the level of synchrony. The music then may be considered to be the "output which depends on the level of synchrony" output by the output system.

A similar reward-based music feedback loop has been proven to be effective for the use of brain signals to promote relaxation. Apparently, people are (unconsciously) so keen to listen to good quality music that they learn how to adapt their behavior in order to achieve this. In this example, the feedback is used to promote behavior which improves the synchrony between the adult and child them, and hence improves the attachment relationship.

In a practical use case, the adult, e.g. parent, could sit on the couch with the child on their lap. Heart rate signals, measured with wearables, are sent to the parent's portable device. An application streams the parent's favorite music - or a typical children's song - and slightly degrades the music quality whenever synchrony is low. The lower music quality is an (unconscious) incentive to the parent to adapt the interaction with their child.

The parent and child will find out which types of interaction work for them, and are guided when trying out new ones as the child grows older.

The musical degradation may be intended only for the adult, but it may also target the child, for example by degrading high musical frequencies.

Instead of a music quality, a type of music may be selected, for example some music may be soothing and other music may be more alarming. Thus, the music may be adapted based on an expected level of enjoyment of the particular type of music.

The same approach may be performed based on story telling instead of music. Then parent and child listen together to a story that is being told by a prerecorded voice. The adaptation to the story telling then involves changing the quality/timbre of the voice that tells the story. The story telling output then reflects the level of synchrony to some extent, as the characteristics of the story telling voice are adapted in dependence on the level of synchrony. The story telling audio then may be considered to be the "output which depends on the level of synchrony" provided by the output system.

This feedback approach may use other feedback media, such as room illumination patterns or the creation of pleasant images, again with degraded quality in response to low synchrony.

### Example 4

Many of the physiological signals that can be used for the synchrony measurement, e.g. heart rate or skin conductance, are also reflective of someone's arousal or stress or anxiety level. This can be particularly informative in the situation in which the child is distressed and needs comforting. The physiological measures of both parent and child may be analyzed in terms of anxiety and stress, and the system may then monitor them while the parent is comforting the child.

The measures are used as an indication as to whether the parent's comforting actions are successful. In that case, the physiological signals of the child (heart rate, skin conductivity) will gradually drop. At the same time, the behavioral and physiological synchrony of adult and child is monitored to be able to indicate to the adult over time how the attachment relationship is developing, since this is linked to moments of distress and a baby turning to the adult for comforting.

Firstly, the cry or discomfort of the baby is recognized which can be achieved with various technologies: wearable baby sensors (HR/respiration/movement), camera (movement and/or facial expression), microphone (sound/cry detection), as part of a baby monitoring system.

Secondly, the parent is alerted to this cry or discomfort, and based on its type, is advised which comforting method is best, taking their own historical data into account, as well as data from a larger data set of comparable children and contexts (e.g. developmental age, time of day, type of cry).

If the stress level of the parent is too high, the system advises the parent to first reduce their own stress level before they help the baby, e.g. 'take a few calming breaths'.

Thirdly, the levels of anxiety and synchrony of the parent and child are monitored while the parent is comforting the child. This can be done with wearable sensors or cameras, microphones etc. After the comforting, the anxiety and synchrony measurements give an indication of success of the comforting action and the development of the attachment relationship.

The system may detect when optimal stress reduction routines are changing as the child develops over time: what might have worked 3 months ago, might not be so effective anymore now. The system can also give practical tips to reduce the child's (and parent's) anxiety level. The parent could also deliberately choose the most effective technique in stressful situations for the child (e.g. during vaccination injections) based on the feedback.

### Example 5

Kangaroo care is an existing method of holding a baby in such a way that it involves skin-to-skin contact. The baby, who is typically naked except for a diaper, is placed in an upright position against a parent's bare chest. Kangaroo care can help the baby to regulate their temperature or circadian rhythm. In this way, the parent co-regulates their child. Also, the skin-to-skin contact that is involved helps the development of the bond between the parent and child. As both mothers and fathers can do kangaroo care it can help not only the mother but also the father to bond with their child.

This example for example makes use of a baby carrier that can be used for kangarooing. It is enhanced with sensors that measure the physiological responses such as heart rate. The parent can then receive real-time feedback about the level of adult-child synchrony, as an indication of the co-regulation processes that are the building blocks of attachment. When there is little synchrony, parents may then be provided with advice that helps them to improve their synchrony. For example, a piece of advice could be that the parent slowly rub their infant's back or quietly sing to their baby, to enhance the contact between them. This baby carrier can be used for newborns as well as babies which are a few months old in different situations when the mother or father is carrying their baby.

### General

It is discussed above that the synchrony level may be output to the user. A transformation of the calculated synchrony level may be performed to translate the synchrony level into an attachment-support-score. An exponential transformation for example may emphasize that high synchrony levels contribute much more to the build-up of the attachment relationship than medium levels. Or, reversely, it might be the case that the optimal transformation of the synchrony levels might be a compression, reflecting that high synchrony levels contribute only slightly (or not even) more to the build-up of the attachment relationship than the medium synchrony levels.

By providing historical data to an external database (in the cloud), an overall population expected synchrony level curve can be constructed for each activity as a function of the age of the infant. This data can then be used to predict the synchrony level over time and as a basis for activity proposals.

The synchrony measurements may be incorporated into a game between parents, or between families, in order to see who can achieve the highest synchrony level. This game will be beneficial to build up good attachment relationship between the child and each competing adult.

The use of feedback, for example when a current activity or interaction results in high synchrony, enables parents to emphasize and continue the activity or fine tune the activity.

The output may provide more detailed analysis, for example the system may automatically record, recognize and label the adult-child interaction via a smart camera and plot the synchrony levels over the timeline of the video. There may be some people for whom the system may not work, such as adults whose medication influences heart rate variations. The system may then indicate for which parents this application is helpful, for instance excluding certain parents for which it might not work.

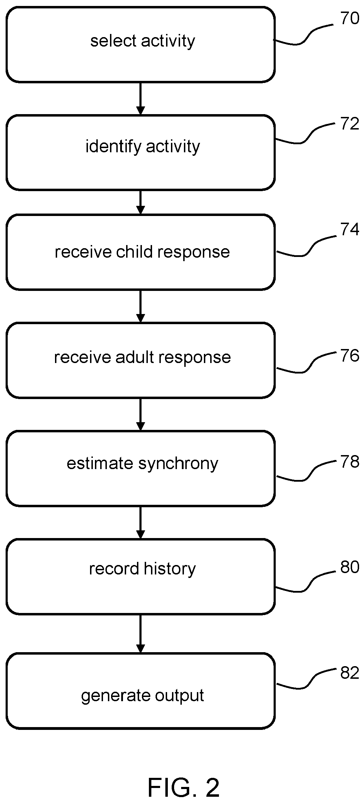

Figure 2 shows a computer-implemented method for assisting the development of an adult-child attachment relationship, comprising:
in step 70 selecting one of a set of possible adult-child activities from a memory storing a set of possible adult-child interactive activities or receiving an indication of a user-selected adult-child activity;
in step 72, if the activity has been selected by the method, controlling an output system to identify the selected adult-child activity (so that the adult knows what activity to perform);
in step 74, receiving one or more sensed child physiological or behavioral signals;
in step 76, receiving or more adult physiological or behavioral signals;
in step 78, analyzing an association between the adult physiological or behavioral signals and the child physiological or behavioral signals thereby to estimate a level of synchrony between the adult and child associated with the selected adult-child activity; and
in step 82, controlling the output system to provide an output which depends on the level of synchrony.

Selecting one of a set of possible adult-child activities may further take account of historical data relating to the use of the adult-child activities and the method comprises recording the estimated level of synchrony as historical data in step 80.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for assisting in the development of an adult-child attachment relationship, comprising:
a physiological or behavioral sensor arrangement (10,20,50,54) for sensing one or more child physiological or behavioral signals and for sensing one or more adult physiological or behavioral signals;
an output system (40); and
a controller (42),
wherein the controller (42) is adapted to:
for one of a set of possible adult-child activities, analyze an association between the adult physiological or behavioral signals and the child physiological or behavioral signals, and thereby estimate a level of synchrony between the adult and child associated with the selected adult-child activity; and
control the output system (40) to provide an output which depends on the level of synchrony.

2. The system of claim 1, wherein the controller is adapted to:
select said one of the set of possible adult-child activities and control the output system to identify a selected adult-child activity to be performed; or
receive an indication of a user-selected adult-child activity;

3. The system of claim 1 or 2, comprising:
a memory (60), or a communications system for interrogating an external memory, storing the set of possible adult-child interactive activities and historical data relating to the use of the adult-child interactive activities;
wherein the controller (42) is adapted to select one of a set of possible adult-child activities based in part on the historical data and to control the memory to record the level of synchrony as historical data.

4. The system of any one of claims 1 to 3, wherein the child and adult physiological or behavioral signals each comprise or identify one or more of:
a heart rate;
a skin conductance;
respiration characteristics;
a direction of gaze;
a type of facial expression;
sounds made by the child and/or adult;
movements.

5. The system of any one of claims 1 to 4, wherein the physiological or behavioral sensor arrangement comprises a microphone (54) for recording sounds made during adult-child interaction.

6. The system of any one of claims 1 to 5, wherein the controller (42) is adapted to derive a level of anxiety of the child from the child physiological or behavioral signal and optionally also a level of anxiety of the adult from the adult physiological or behavioral signal.

7. The system of any one of claims 1 to 6, wherein the controller (42) is adapted to provide a feedback signal which depends on the estimated level of synchrony, wherein the feedback signal is for identifying suitable activities for improving the level of synchrony.

8. The system of any one of claims 1 to 7, comprising:
a speaker (52) for playing music, wherein the controller is adapted to select characteristics of the music in dependence on the estimated level of synchrony during the adult-child activity, such that the music output reflects the level of synchrony to some extent; or
a speaker (52) for playing a story telling, wherein the controller is adapted to select the characteristics of the story telling voice in dependence on estimated level of synchrony, such that the story telling voice reflects the level of synchrony to some extent.

9. The system of any one of claims 1 to 8, wherein the set of adult-child activities include one or more of:
a peek-a-boo game;
a sound copying game;
a Kangaroo care session;
the adult singing to the child;
the adult rubbing the child's back;
bedtime rituals;
visiting a venue for an interactive experience; and
performing arts and crafts together.

10. The system of any one of claims 1 to 9, wherein the controller (42) is adapted to:
receive an identification of an adult-child activity to add to the set; or
request identification of an adult-child activity based on continuous monitoring of the synchrony level.

11. A computer-implemented method for assisting the development of an adult-child attachment relationship, comprising:
(74) during one of a set of possible adult-child activities, receiving one or more sensed child physiological or behavioral signals;
(76) receiving or more adult physiological or behavioral signals;
(78) analyzing an association between the adult physiological or behavioral signals and the child physiological or behavioral signals, and thereby estimating a level of synchrony between the adult and child associated with the selected adult-child activity; and
(82) controlling the output system to provide an output which depends on the level of synchrony.

12. The method of claim 11 wherein selecting one of a set of possible adult-child activities further takes account of historical data relating to the use of the adult-child activities and the method comprises (80) recording the estimated level of synchrony as historical data.

13. The method of claim 11 or 12, wherein:
the child physiological or behavioral signals and adult physiological or behavioral signals responses each comprise or identify one or more of:
a heart rate;
a skin conductance;
respiration characteristics;
a direction of gaze;
a type of facial expression; and
movements; and/or
wherein the set of adult-child activities include one or more of:
a peek-a-boo game;
a sound copying game;
a Kangaroo care session;
the adult singing to the child;
the adult rubbing the child's back;
bedtime rituals;
visiting a venue for an interactive experience; and
performing arts and crafts together.

14. A computer program comprising computer program code means which is adapted, when said program is run on the controller of the system of any one of claims 1 to 10, to implement the method of any one of claims 11 to 13.

15. A controller configured to run the computer program of claim 14.
